# FASCICULE DE BREVET EUROPEEN

(11) **EP 1 270 562 B1**
(45) Date de publication et mention de la délivrance du brevet: **22.09.2004**
(21) Numéro de dépôt: 02291501.1
(22) Date de dépôt: 17.06.2002
(51) Int. Cl.: C07D 295/18, C07C 237/14, A61K 31/16, A61K 31/40, A61P 3/00, C07D 205/04, C07D 209/02, C07D 277/22, C07D 207/20

(54) **Nouveaux dérivés cycliques d'alpha-amino-gamma-hydroxy-amides, leur procédé de préparation et les compositions pharmaceutiques qui les contiennent**
Cyclische Alpha-amino-gamma-hydroxy Säurederivate und diese Verbindungen enthaltende pharmazeutische Mittel
Cyclic alpha-amino-gamma-hydroxy-amides, process for their preparation and pharmaceutical compositions thereof

(30) Priorité: 18.06.2001 FR 0107934
(43) Date de publication de la demande: 02.01.2003
(73) Titulaire: Les Laboratoires Servier, 92200 Neuilly-sur-Seine (FR)
(72) Inventeur: Wierzbicki, Michel, 78620 L'Etang la Ville (FR); Fourquez, Jean-Marie, 92150 Suresnes (FR); Levens, Nigel, 92420 Vaucresson (FR); Husson-Robert, Bernadette, 92000 Nanterre (FR); Nosjean, Olivier, 92500 Rueil Malmaison (FR); Boulanger, Michelle, 78400 Chatou (FR)

(56) Documents cités:
- WO-A-97/40832
- KATAGIRI N ET AL: "A NEW SYNTHESIS OF HIGHLY FUNCTIONAL NITRONES THROUGH A NITROSOKETENE INTERMEDIATE AND THEIR USE FOR THE STEREOSELECTIVE SYNTHESIS OF AMINO ACIDS" JOURNAL OF ORGANIC CHEMISTRY, AMERICAN CHEMICAL SOCIETY. EASTON, US, vol. 59, no. 26, 1994, pages 8101-8106, XP002021582 ISSN: 0022-3263

## Description

La présente invention concerne de nouveaux dérivés cycliques d'α-amino-γ-hydroxyamides, leur procédé de préparation, les compositions pharmaceutiques qui les contiennent ainsi que leur utilisation en tant qu'antidiabétiques.

WO 9740832 décrit l'utilisation d'agents réducteurs de l'activité de la peptidase dipeptidyl IV pour le traitement de maladies qui sont fondées sur une concentration de glucose.

Un dérivé cyclique d'α-amino-γ-hydroxy-acide a été décrit dans le journal J. Org. Chem. 1994, 59 (26), 8101-6, sans qu'aucune activité pharmacologique n'ait été citée pour ce composé.

Plus spécifiquement, la présente invention concerne les composés de formule (I) : 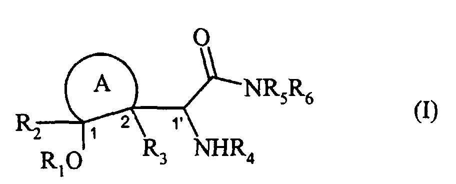 dans laquelle : 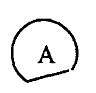
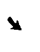 représente un cycle carboné saturé de 4 à 8 chaînons, éventuellement substitué par un ou plusieurs groupements alkyle (C₁-C₆) linéaire ou ramifié,
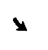 R₁ et R₄, identiques ou différents, représentent chacun un atome d'hydrogène ou un groupement acyle (C₁-C₆) linéaire ou ramifié,
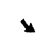 R₂ et R₃, identiques ou différents, représentent chacun un atome d'hydrogène ou un groupement alkyle (C₁-C₆) linéaire ou ramifié,
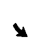 R₅ et R₆, identiques ou différents, représentent chacun un atome d'hydrogène ou un groupement alkyle (C₁-C₆) linéaire ou ramifié,
   ou bien R₅ et R₆ forment ensemble, avec l'atome d'azote qui les porte, un hétérocycle azoté éventuellement substitué par un ou plusieurs groupements, identiques ou différents, choisis parmi les groupements cyano, CO₂R₇ (dans lequel R₇ représente un atome d'hydrogène ou un groupement alkyle (C₁-C₆) linéaire ou ramifié), COR₇ (dans lequel R₇ est tel que défini précédemment), nitro, CONR₈ₐR_{8b} (dans lequel R₈ₐ et R_{8b}, identiques ou différents, représentent chacun un atome d'hydrogène ou un groupement alkyle (C₁-C₆) linéaire ou ramifié, ou bien R₈ₐ et R_{8b} forment ensemble un hétérocycle azoté), S(O)ₙR₉ (dans lequel n représente 1, 2 ou 3, et R₉ représente un atome d'hydrogène, un groupement alkyle (C₁-C₆) linéaire ou ramifié ou aryle), ou PO₃R₁₀ₐR_{10b} (dans lequel R₁₀ₐ et R_{10b}, identiques ou différents, représentent chacun un atome d'hydrogène ou un groupement alkyle (C₁-C₆) linéaire ou ramifié),
leurs stéréoisomères, ainsi que leurs sels d'addition à un acide pharmaceutiquement acceptable.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique, camphorique.

Par hétérocycle azoté, on entend un groupement saturé, mono- ou bicyclique, éventuellement ponté, comportant de 5 à 12 chaînons et contenant un, deux ou trois hétéroatomes, l'un de ces hétéroatomes étant l'atome d'azote, et le ou les hétéroatomes supplémentaires éventuellement présents étant choisis parmi les atomes d'oxygène, d'azote ou de soufre. Les hétérocycles azotés préférés sont les groupements pyrrolidinyle, thiazolidinyle, pipéridyle, morpholinyle, azabicyclo[3.3.0]octyle et pipérazinyle.

Par groupement aryle, on entend phényle, biphénylyle, naphtyle, ou tétrahydronaphtyle, chacun de ces groupements étant éventuellement substitué par un ou plusieurs atomes ou groupements, identiques ou différents, choisis parmi les atomes d'halogène et les groupements alkyle (C₁-C₆) linéaire ou ramifié, hydroxy, alkoxy (C₁-C₆) linéaire ou ramifié, polyhalogénoalkyle (C₁-C₆) linéaire ou ramifié, nitro ou alkylènedioxy (C₁-C₂).

Les composés préférés de formule (I) sont ceux pour lesquels 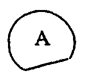 représente un cycle carboné de 5 ou 6 chaînons.

Un aspect avantageux de l'invention concerne les composés de formule (I) pour lesquels R₅ et R₆ forment ensemble un hétérocycle azoté éventuellement substitué.
Parmi ceux-ci, on préférera tout particulièrement ceux pour lesquels R₅ et R₆ forment ensemble une pyrrolidine ou une thiazolidine éventuellement substituées.

Parmi les composés préférés de l'invention, on peut citer plus particulièrement :
- le (1R,2S,1'S)-2-[1'-amino-2'-oxo-2'-(1-pyrrolidinyl)-éthyl]-cyclohexanol, son énantiomère (1S,2R,1'R), ainsi que leurs sels d'addition à un acide pharmaceutiquement acceptable;
- le (1R*,2R*,1'R*)-2-[1'-amino-2'-oxo-2'-(1-pyrrolidinyl)-éthyl]-cyclopentanol, ainsi que ses sels d'addition à un acide pharmaceutiquement acceptable, étant entendu que par composé (1R*,2R*,1'R*), on entend mélange racémique des 2 énantiomères de configurations absolues (1R,2R,1'R) et (1S,2S,1'S);
- le (1R,2S,1'R)-2-[1'-amino-2'-oxo-2'-(1-pyrrolidinyl)-éthyl]-cyclopentanol, son énantiomère (1S,2R,1'S), ainsi que leurs sels d'addition à un acide pharmaceutiquement acceptable;
- le (1R,2S,1'S)-2-[1'-amino-2'-oxo-2'-(1-pyrrolidinyl)-éthyl]-cyclopentanol, son énantiomère (1S,2R,1'R), ainsi que leurs sels d'addition à un acide pharmaceutiquement acceptable;
- le (1R,2S,1'R)-2-[1'-amino-2'-oxo-2'-(1-pyrrolidinyl)-éthyl]-cyclohexanol, son énantiomère (1S,2R,1'S), ainsi que leurs sels d'addition à un acide pharmaceutiquement acceptable;
- et le (1R,2S,1'R)-2-[1'-amino-2'-oxo-2'-(1,3-thiazolidin-3-yl)-éthyl]-cyclohexanol, son énantiomère (1S,2R,1'S), ainsi que leurs sels d'addition à un acide pharmaceutiquement acceptable.

L'invention s'étend également à un procédé de préparation des composés de formule (I), caractérisé en ce que l'on met en réaction le chlorure de chloroacétyle avec le composé de formule (II) :

HNR₅R₆ (II)

dans laquelle R₅ et R₆ sont tels que définis dans la formule (I), pour conduire au composé de formule (III) : 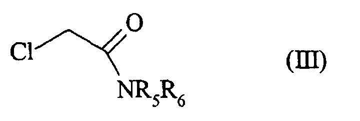 dans laquelle R₅ et R₆ sont tels que définis précédemment, que l'on transforme en amine de formule (IV) : 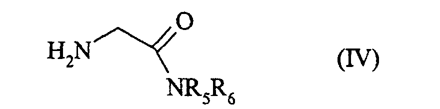 dans laquelle R₅ et R₆ sont tels que définis précédemment, que l'on met en réaction avec le benzaldéhyde, pour conduire au composé de formule (V) : 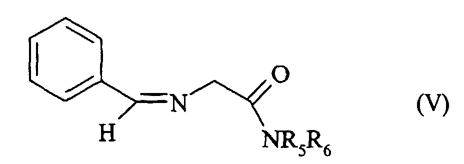 dans laquelle R₅ et R₆ sont tels que définis précédemment, que l'on met en réaction avec le composé de formule (VI) : 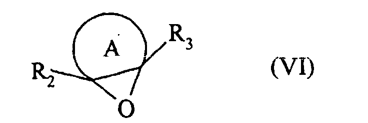 dans laquelle 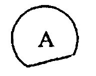 , R₂ et R₃ sont tels que définis dans la formule (I),
pour conduire après acylation éventuelle de la fonction hydroxy au composé de formule (VII), qui est de configuration relative trans : 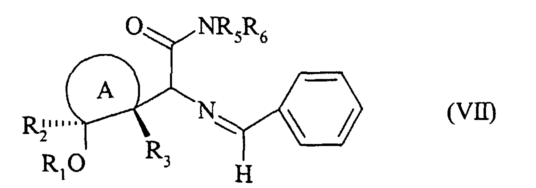 dans laquelle 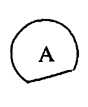 , R₁, R₂, R₃, R₅ et R₆ sont tels que définis précédemment,
que l'on transforme, par hydrolyse suivie, lorsqu'on le souhaite, d'une acylation de la fonction amino, au composé de formule (Ia) de configuration relative trans, cas particulier des composés de formule (I) : 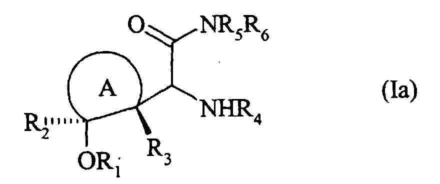 dans laquelle 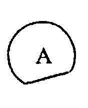 , R₁, R₂, R₃, R₄, R₅ et R₆ sont tels que définis précédemment,
que l'on transforme, lorsqu'on le souhaite, en composé de formule (Ib) de configuration relative cis, cas particulier des composés de formule (I) : 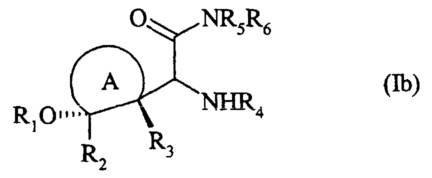 dans laquelle 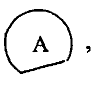 , R₁, R₂, R₃, R₄, R₅ et R₆, sont tels que définis précédemment,
composés de formules (Ia) et (Ib) dont on sépare les stéréoisomères, lorsqu'on le souhaite, par des techniques classiques de séparation, que l'on purifie, le cas échéant, par des méthodes classiques de purification, et que l'on transforme, lorsqu'on le souhaite, en leurs sels d'addition à un acide pharmaceutiquement acceptable.

Les composés de formule (Ic), cas particulier des composés de formule (I) : 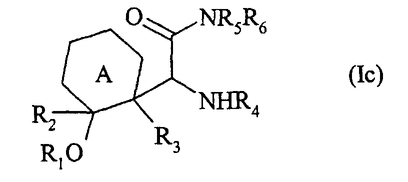 dans laquelle 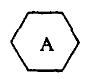 représente un cycle carboné saturé de 6 chaînons éventuellement substitué par un ou plusieurs groupements alkyle (C₁-C₆) linéaire ou ramifié, et R₁, R₂, R₃, R₄, R₅ et R₆ sont tels que définis dans la formule (I),
peuvent également être obtenus à partir du composé de formule (VIII) : 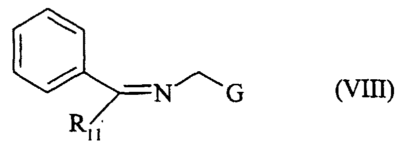 dans laquelle R₁₁ représente un atome d'hydrogène ou un groupement phényle, et G représente CN ou un groupement alkoxycarbonyle (C₁-C₆) linéaire ou ramifié,
que l'on met en réaction avec le composé de formule (VIa), cas particulier des composés de formule (VI) : 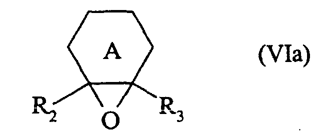 dans laquelle 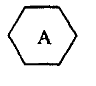 , R₂ et R₃ sont tels que définis précédemment,
pour conduire au composé de formule (IX) de configuration relative trans : 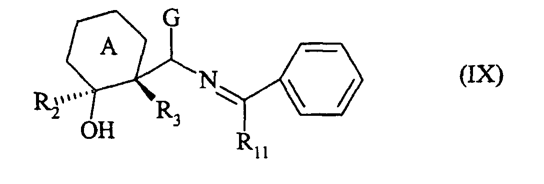 dans laquelle 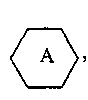 , G, R₂, R₃ et R₁₁ sont tels que définis précédemment,
composé de formule (IX) :
- que l'on hydrolyse dans des conditions acides, lorsqu'on souhaite parvenir à un composé de configuration relative trans, pour conduire, après acylation éventuelle de la fonction amino, au composé de formule (Xt) dont la jonction de cycle est trans : 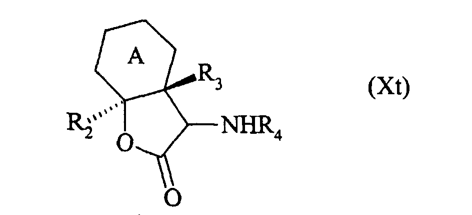 dans laquelle 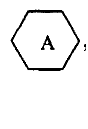 , R₂, R₃ et R₄ sont tels que définis précédemment,
   que l'on met en réaction avec le composé de formule (II), pour conduire, après acylation éventuelle de la fonction hydroxy, au composé de formule (Id) de configuration relative trans, cas particulier des composés de formule (Ic) : 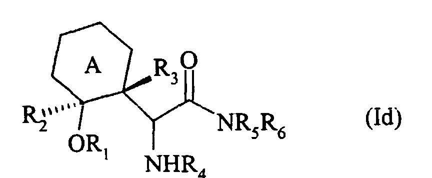 dans laquelle 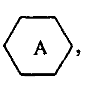 , R₁, R₂, R₃, R₄, R₅ et R₆ sont tels que définis précédemment,
- ou que l'on soumet à une réaction de Mitsunobu, lorsqu'on souhaite parvenir à un composé de configuration relative cis, pour conduire, après acylation éventuelle de la fonction amino, au composé de formule (Xc) dont la jonction de cycle est cis : 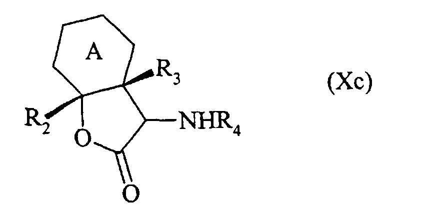
dans laquelle 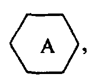 , R₂, R₃ et R₄ sont tels que définis précédemment,
que l'on met en réaction avec le composé de formule (II), pour conduire, après acylation éventuelle de la fonction hydroxy, au composé de formule (Ie) de configuration relative cis, cas particulier des composés de formule (Ic): 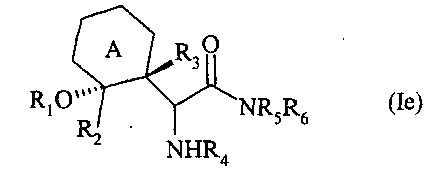 dans laquelle 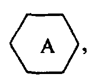 R₁, R₂, R₃, R₄, R₅ et R₆ sont tels que définis précédemment,
composés de formules (Id) et (Ie) dont on sépare les stéréoisomères, lorsqu'on le souhaite, par des techniques classiques de séparation, que l'on purifie, le cas échéant, par des méthodes classiques de purification, et que l'on transforme, lorsqu'on le souhaite, en leurs sels d'addition à un acide pharmaceutiquement acceptable.

Les composés de formule (If), de configuration relative cis, cas particulier des composés de formule (I) : 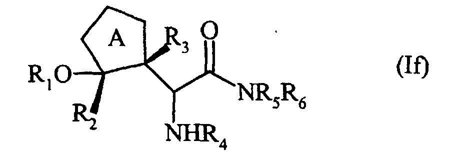 dans laquelle 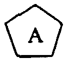 représente un cycle carboné saturé de 5 chaînons éventuellement substitué par un ou plusieurs groupements choisis parmi alkyle (C₁-C₆) linéaire ou ramifié, et R₁, R₂, R₃, R_{4,} R₅ et R₆ sont tels que définis dans la formule (I), peuvent également être obtenus à partir du composé de formule (VIII) que l'on met en réaction avec le composé de formule (VIb), cas particulier des composés de formule (VI): 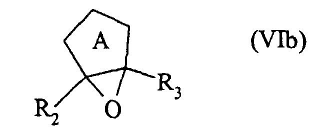 dans laquelle 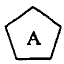 , R₂ et R₃ sont tels que définis précédemment,
pour conduire au composé de formule (XI) de configuration relative trans : 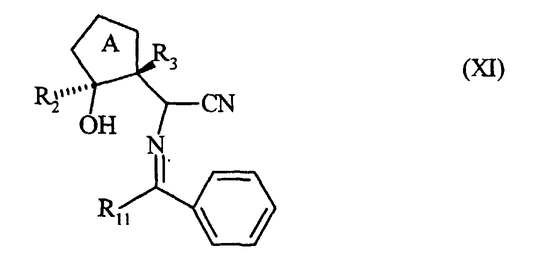 dans laquelle 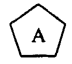 , R₂, R₃ et R₁₁ sont tels que définis précédemment,
que l'on hydrolyse dans des conditions acides, pour conduire, après acylation éventuelle de la fonction amino, au composé de formule (XII) dont la jonction de cycle est cis : 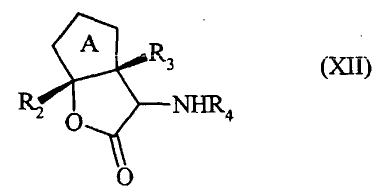 dans laquelle 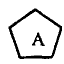 , R₂, R₃ et R₄ sont tels que définis précédemment,
que l'on met en réaction avec le composé de formule (II), pour conduire, après acylation éventuelle de la fonction hydroxy, au composé de formule (If), dont on sépare les stéréoisomères, lorsqu'on le souhaite, par des techniques classiques de séparation, que l'on purifie, le cas échéant, par des méthodes classiques de purification, et que l'on transforme, lorsqu'on le souhaite, en leurs sels d'addition à un acide pharmaceutiquement acceptable.

Les composés de formule (Ig), dé configuration relative trans, cas particulier des composés de formule (I) : 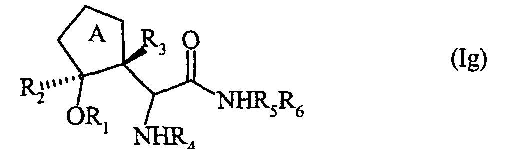 peuvent également être obtenus à partir du composé de formule (XIII) : 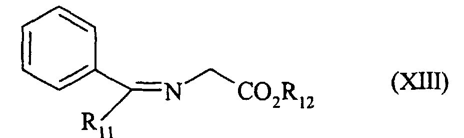 dans laquelle R₁₁ est tel que défini précédemment, et R₁₂ représente un groupement alkyle (C₁-C₆) linéaire ou ramifié,
que l'on met en réaction avec le composé de formule (VIb), pour conduire au composé de formule (XIV) de configuration relative trans : 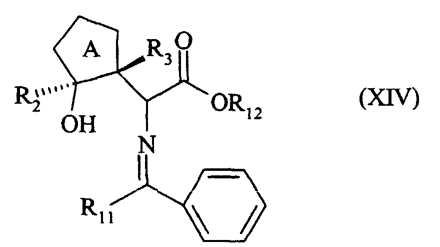 dans laquelle 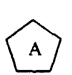 , R₂, R₃, R₁₁ et R₁₂ sont tels que définis précédemment,
que l'on hydrolyse dans des conditions acides douces, pour conduire, après acylation ou protection de la fonction amino, au composé de formule (XV) de configuration relative trans : 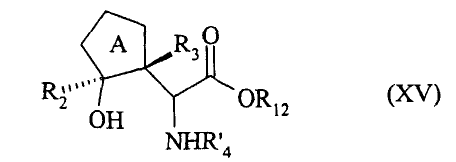 dans laquelle 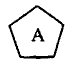 , R₂, R₃ et R₁₂ sont tels que définis précédemment, et R'₄ représente, soit un groupement alkyle (C₁-C₆) linéaire ou ramifié, soit un groupement protecteur de la fonction amino,
que l'on met en réaction, dans des conditions de couplage peptidique, avec le composé de formule (II), pour conduire, après acylation éventuelle de la fonction hydroxy et déprotection éventuelle de la fonction amino, au composé de formule (Ig), dont on sépare les stéréoisomères, lorsqu'on le souhaite, par des techniques classiques de séparation, que l'on purifie, le cas échéant, par des méthodes classiques de purification, et que l'on transforme, lorsqu'on le souhaite, en leurs sels d'addition à un acide pharmaceutiquement acceptable.

Les composés de la présente invention, outre le fait qu'ils soient nouveaux, présentent des propriétés pharmacologiques intéressantes. Ils ont des propriétés hypoglycémiantes qui les rendent utiles dans le traitement de l'intolérance au glucose et des maladies associées à une hyperglycémie telles que le diabète de type II ou l'obésité.

L'invention s'étend aussi aux compositions pharmaceutiques renfermant comme principe actif au moins un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse, intramusculaire ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables, etc.

La posologie utile est adaptable selon la nature et la sévérité de l'affection, la voie d'administration ainsi que l'âge et le poids du patient et les traitements éventuellement associés. Cette posologie varie de 0,5 mg à 2 g par 24 heures en une ou plusieurs prises.

Les exemples suivants illustrent l'invention et ne la limitent en aucune façon.

Les produits de départ utilisés sont des produits connus ou préparés selon des modes préparatoires connus.

Les structures des composés décrits dans les exemples ont été déterminées selon les techniques spectrométriques usuelles (infrarouge, RMN, spectrométrie de masse).

Par composé (1R*,2R*,1'S*), on entend mélange racémique des 2 énantiomères de configurations absolues (1R,2R,1'S) et (1S,2S,1'R).

Par composé (1R*,2R*,1'RS), on entend mélange des 4 stéréoisomères de configurations absolues (1R,2R,1'R), (1R,2R,1'S), (1S,2S,1'R) et (1S,2S,1'S).

Par composé de configuration (1α,2β,1'β) ou (1β,2α,1'α), on entend composé choisi parmi les composés de configurations absolues (1R,2S,1'S) et (1S,2R,1'R), étant entendu que lorsque le composé (1α,2β,1'β) représente le composé de configuration (1R,2S,1'S), alors le composé (1β,2α,1'α) représente l'autre énantiomère.

Par fumarate d'un composé, on entend sel d'addition 1:1 dudit composé avec l'acide fumarique (1 mole d'acide fumarique par mole de composé).
Par hémifumarate d'un composé, on entend sel d'addition 1:0,5 dudit composé avec l'acide fumarique (0,5 mole d'acide fumarique par mole de composé).

### EXEMPLE 1 : (1R*,2S*,1'S*)-2-[1'-Amino-2'-oxo-2'-(1-pyrrolidinyl)-éthyl]-cyclohexanol, fumarate

### Stade A : (2RS,1'R*,2'S*)-2-[(Diphénylméthylène)-amino]-2-(2'-hydroxycyclohexyl)-acétonitrile

A 10 mmoles de diisopropylamine en solution dans le tétrahydrofurane à -30/-40°C sont ajoutées 10 mmoles d'une solution 2,5 M de n-butyllithium, puis, après 15 minutes d'agitation à cette température, le milieu réactionnel est refroidi à -70°C et 10 mmoles de [(diphénylméthylène)-amino]-acétonitrile en solution dans le tétrahydrofurane sont ajoutées. Après 4 heures d'agitation à -70°C, 10 mmoles d'oxyde de cyclohexène dans le tétrahydrofurane sont ajoutées, puis 10 mmoles d'éthérate de trifluorure de bore. Le mélange réactionnel est agité 1 heure supplémentaire à -70°C, puis il est ramené à +10°C avant d'être hydrolysé.

### Stade B : (3R*,3aR*,7aS*)-3-Amino-hexahydro-1-benzofuran-2(3H)-one

Au mélange brut obtenu au stade précédent sont ajoutés 160 ml d'acide chlorhydrique 4M. Le mélange réactionnel est alors agité pendant 3 jours, puis décanté. La phase aqueuse est lavée à l'éther diéthylique, puis 25 ml d'acide chlorhydrique concentré sont ajoutés et le mélange réactionnel est chauffé 5 jours à 60°C. Après retour à température ambiante, les solvants sont évaporés. Au résidu obtenu est ajoutée de l'eau, puis du carbonate de potassium en quantité suffisante pour atteindre un pH de 9-10. Après extraction par le dichlorométhane, les phases organiques rassemblées sont séchées, filtrées puis évaporées. Le résidu obtenu est purifié par chromatographie sur silice (éluant: dichlorométhane/méthanol 95/5), puis les diastéréoisomères sont séparés par HPLC préparative. Le produit attendu est le premier des diastéréoisomères ainsi séparés.

### Stade C : (1R*,2S*,1'S*)-2-[1'-Amino-2'-oxo-2'-(1-pyrrolidinyl)-éthyl]-cyclohexanol

A 10 mmoles du composé obtenu au stade précédent en solution dans le toluène sont ajoutées 20 mmoles de pyrrolidine. Après 24 heures d'agitation à température ambiante, le solvant est évaporé. Le résidu obtenu est purifié par chromatographie sur silice (éluant : dichlorométhane/méthanol/ammoniaque 90/10/1) pour conduire au produit attendu sous la forme d'un mélange racémique.

### Stade D : (1R*,2S*,1'S*)-2-[1'-Amino-2'-oxo-2'-(1-pyrrolidinyl)-éthyl]-cyclohexanol, fumarate :

Le composé obtenu au stade précédent est transformé en son fumarate par réaction avec une solution éthanolique d'acide fumarique, filtration puis séchage, pour conduire au produit attendu.

### EXEMPLE 2A : (1α,2β,1'β)-2-[1'-Amino-2'-oxo-2'-(1-pyrrolidinyl)-éthyl]-cyclohexanol, fumarate

Le mélange racémique obtenu au stade C de l'exemple 1 est séparé par chromatographie HPLC chirale préparative (colonne : Chiralpack AS, éluant : n-heptane/isopropanol/diéthylamine). Le premier des énantiomères obtenus est transformé en son fumarate par réaction avec une solution éthanolique d'acide fumarique, filtration puis séchage, pour conduire au produit attendu sous la forme d'un solide blanc.
*Point de fusion : 190-192°C*
*Pouvoir rotatoire :α*_{*D*} *= -20,23° (eau, c=1, λ = 365 nm)*

| *Microanalyse élémentaire :* | | | |
|---|---|---|---|
| | *C%* | *H%* | *N%* |
| *calculé* | *56,13* | *7,65* | *8,18* |
| *trouvé* | *56,13* | *7,74* | *8,05* |

### EXEMPLE 2B : (1β,2α,1'α)-2-[1'-Amino-2'-oxo-2'-(1-pyrrolidinyl)-éthyl]-cyclohexanol, fumarate

Le produit attendu est obtenu par transformation en fumarate du deuxième des énantiomères obtenus à l'exemple 2A.
*Point de fusion : 190°C*

| *Microanalyse élémentaire :* | | | |
|---|---|---|---|
| | *C%* | *H%* | *N%* |
| *calculé* | *56,13* | *7,65* | *8,18* |
| *trouvé* | *56,08* | *7,71* | *8,06* |

### EXEMPLE 3 : (1R*,2R*,1'R*)-2-[1'-Amino-2'-oxo-2'-(1-pyrrolidinyl)-éthyl]-cyclopentanol, fumarate

### Stade A : (1R*,2R*,1'RS)-2-[1'-Amino-2'-oxo-2'-(1-pyrrolidinyl)-éthyl]-cyclopentanol

Le produit attendu est obtenu selon le procédé décrit dans les stades A à C de l'exemple 1, en remplaçant l'oxyde de cyclohexène par l'oxyde de cyclopentène.

### Stade B : (1R*,2R*,1'R*)-2-[1'-Amino-2'-oxo-2'-(1-pyrrolidinyl)-éthyl]-cyclopentanol, fumarate

Le composé obtenu au stade précédent est purifié par chromatographie sur silice (éluant : dichlorométhane/méthanol/ammoniaque 90/10/1). Le premier des diastéréoisomères obtenus, par ordre d'élution, est ensuite transformé en son fumarate pour conduire au produit attendu.
*Point de fusion : 175°C*

| *Microanalyse élémentaire :* | | | |
|---|---|---|---|
| | *C%* | *H%* | *N%* |
| *calculé* | *54,87* | *7,37* | *8,53* |
| *trouvé* | *55,14* | *7,56* | *8,48* |

### EXEMPLE 4 : (1R*,2R*,1'S*)-2-[1'-Amino-2'-oxo-2'-(1-pyrrotidinyl)-éthyl]-cyclopentanol, hémifumarate

Le produit attendu est obtenu par transformation en hémifumarate du deuxième des diastéréoisomères obtenus au stade B de l'exemple 3.
*Point de fusion : 210°C*

| *Microanalyse élémentaire :* | | | |
|---|---|---|---|
| | *C%* | *H%* | *N%* |
| *calculé* | *57,76* | *8,20* | *10,36* |
| *trouvé* | *57,89* | *8,25* | *10,29* |

### EXEMPLE 5 : (1α,2α,1'α)-2-[1'-Amino-2'-oxo-2'-(1-pyrrolidinyl)-éthyl]-cyclopentanol, fumarate

Le mélange racémique de l'exemple 3 est séparé par chromatographie HPLC chirale préparative. Le premier des énantiomères obtenus est transformé en son fumarate par réaction avec une solution éthanolique d'acide fumarique, filtration puis séchage, pour conduire au produit attendu sous la forme d'un solide blanc.

### EXEMPLE 6 : (1β,2β,1'β)-2-[1'-Amino-2'-oxo-2'-(1-pyrrolidinyl)-éthyl]-cyclopentanol, fumarate

Le produit attendu est obtenu par transformation en fumarate du deuxième des énantiomères obtenus dans l'exemple 5.

### EXEMPLE 7 : (1R*,2S*,1'R*)-2-[1'-Amino-2'-oxo-2'-(1-pyrrolidinyl)-éthyl]-cyclopentanol, hémifumarate

### Stade A : [(Diphénylméthylène)-amino]-(2-hydroxycyclopentyl)-acétate d'éthyle

Le produit attendu est obtenu selon le procédé décrit dans le stade A de l'exemple 1 à partir de N-diphénylméthylène-glycinate d'éthyle et d'oxyde de cyclopentène.

### Stade B : (1R*,2S*,1'RS)-Amino-(2-hydroxycyclopentyl)-acétate d'éthyle

A 10 mmoles du composé obtenu au stade précédent en solution dans l'éther sont ajoutées 24 mmoles d'une solution dans 1M d'acide chlorhydrique. Le mélange réactionnel est ensuite agité pendant 30 minutes à température ambiante, puis décanté. La phase aqueuse est alors lavée à l'éther, puis son pH est amené entre 7 et 8 par addition de carbonate de potassium. Après extraction au dichlorométhane, la phase organique est séchée puis filtrée et le solvant est évaporé pour conduire au produit attendu.

### Stade C : (1R*,2S*,1'R*)-Amino-(2-hydroxycyclopentyl)-acétate d'éthyle

Le résidu obtenu au stade précédent est chromatographié sur silice (éluant : dichlorométhane/méthanol 90/10). Le produit attendu est le premier des 2 diastéréoisomères ainsi séparés.

### Stade D : (1R*,2S*,1'R*)-[(Benzyloxycarbonyl)-amino]-(2-hydroxycyclopentyl)-acétate d'éthyle

A 10 mmoles du composé obtenu au stade précédent en solution dans l'acétate d'éthyle est ajoutée une solution saturée d'hydrogénocarbonate de sodium, puis, au goutte à goutte, 10 mmoles de chloroformiate de benzyle. Après 2 heures d'agitation à température ambiante, le mélange réactionnel est décanté, la phase aqueuse est extraite à l'acétate d'éthyle puis les phases organiques rassemblées sont lavées, séchées, filtrées, puis évaporées. Le résidu obtenu est purifié par chromatographie sur silice (éluant : dichlorométhane/méthanol 95/5) pour conduire au produit attendu.

### Stade E : Acide (1R*,2S*,1'R*)-[(benzyloxycarbonyl)-amino]-(2-hydroxycyclopentyl)-acétique

A 10 mmoles du composé obtenu au stade précédent en solution dans l'éthanol sont ajoutées 10 mmoles d'une solution 1M de soude. Le mélange réactionnel est ensuite porté au reflux pendant 1 heure, puis l'éthanol est évaporé, au résidu obtenu est ajouté de l'eau, puis 10 mmoles d'acide citrique. La phase aqueuse est alors extraite au dichlorométhane, puis séchée, filtrée et concentrée pour conduire au produit attendu.

### Stade F : (1R*,2S*,1'R*)-2-[1'-[(Benzyloxycarbonyl)-amino]-2'-oxo-2'-(1-pyrrolidinyl)-éthyl]-cyclopentanol

A 10 mmoles du composé obtenu au stade précédent en solution dans le tétrahydrofurane sont ajoutées 12 mmoles de pyrrolidine et 20 mmoles de diisopropyléthylamine, puis un mélange de 12 mmoles de 1-hydroxybenzotriazole et de 12 mmoles de 2-(1*H*-benzotriazol-1-yl)-1,1,3,3-tétraméthyluroniumtétrafluoroborate. Le mélange réactionnel est ensuite agité à température ambiante pendant une nuit, puis de l'acétate d'éthyle est ajouté, et la phase organique est lavée, séchée, filtrée puis concentrée pour conduire au produit attendu.

### Stade G : (1R*,2S*,1'R*)-2-[1'-Amino-2'-oxo-2'-(1-pyrrolidinyl)-éthyl]-cyclopentanol, hémifumarate

A 10 mmoles du composé obtenu au stade précédent en solution dans l'éthanol sont ajoutés 1,7 g de Pd/C à 10 %, puis le mélange est hydrogéné à 90 mbars pendant 16 heures. Après filtration du catalyseur sur célite et lavage à l'éthanol, le filtrat est concentré, puis le résidu obtenu est purifié par chromatographie sur silice (éluant : dichlorométhane/méthanol/NH₄OH 90/10/1). Le produit ainsi obtenu est transformé en son fumarate par addition d'une solution d'acide fumarique dans l'isopropanol, puis cristallisation.
*Point de fusion : 193°C*

| *Microanalyse élémentaire :* | | | |
|---|---|---|---|
| | *C%* | *H%* | *N%* |
| *calculé* | *57,76* | *8,20* | *10,36* |
| *trouvé* | *57,38* | *8,07* | *10,23* |

### EXEMPLE 8 : (1R*,2S*,1'S*)-2-[1'-Amino-2'-oxo-2'-(1-pyrrolidinyl)-éthyl]-cyclopentanol, fumarate

Le produit attendu est obtenu selon le procédé décrit dans les stades D à G de l'exemple 7 à partir du deuxième des diastéréoisomères qui sont séparés au stade C de l'exemple 7. Le produit ainsi obtenu est transformé en son fumarate par addition d'une solution d'acide fumarique dans l'isopropanol, puis cristallisation.
*Point de fusion : 170°C*

| *Microanalyse élémentaire :* | | | |
|---|---|---|---|
| | *C%* | *H%* | *N%* |
| *calculé* | *54,87* | *7,37* | *8,53* |
| *trouvé* | *54,44* | *7,28* | *8,66* |

### EXEMPLE 9A : (1α,2β,1'α)-2-[1'-Amino-2'-oxo-2'-(1-pyrrolidinyl)-éthyl]-cyclopentanol, hémifumarate

Le mélange racémique obtenu au stade G de l'exemple 7 est séparé par chromatographie HPLC chirale préparative sur colonne Chiralpack AD (éluant : n-heptane/éthanol /diéthylamine 75/25/1). Le premier des énantiomères ainsi obtenus est transformé en son hémifumarate par addition d'une solution d'acide fumarique dans l'isopropanol, puis cristallisation.

| *Microanalyse élémentaire :* | | | |
|---|---|---|---|
| | *C%* | *H%* | *N%* |
| *calculé* | *57,76* | *8,20* | *10,36* |
| *trouvé* | *58,08* | *8,13* | *10,11* |

### EXEMPLE 9B : (1α,2β,1'α)-2-[1'-Amino-2'-oxo-2'-(1-pyrrolidinyl)-éthyl]-cyclopentanol, fumarate

Le premier des énantiomères obtenus à l'exemple 9A est transformé en son fumarate par addition d'une solution d'acide fumarique dans l'isopropanol, puis cristallisation. *Point de fusion : 162°C*

| *Microanalyse élémentaire :* | | | |
|---|---|---|---|
| | *C%* | *H%* | *N%* |
| *calculé* | *54,87* | *7,37* | *8,53* |
| *trouvé* | *54,58* | *7,38* | *8,49* |

### EXEMPLE 10 : (1β,2α,1'β)-2-[1'-Amino-2'-oxo-2'-(1-pyrrolidinyl)-éthyl]-cyclopentanol, fumarate

Le deuxième des énantiomères qui sont séparés à l'exemple 9A est transformé en son fumarate par addition d'une solution d'acide fumarique dans l'isopropanol, puis cristallisation.

| *Microanalyse élémentaire :* | | | |
|---|---|---|---|
| | *C%* | *H%* | *N%* |
| *calculé* | *54,87* | *7,37* | *8,53* |
| *trouvé* | *54,97* | *7,43* | *8,41* |

### EXEMPLE 11 : (1α,2β,1'β)-2-[1'-Amino-2'-oxo-2'-(1-pyrrolidinyl)-éthyl]-cyclopentanol, hémifumarate

Le mélange racémique de l'exemple 8 est séparé par chromatographie HPLC chirale préparative. Le premier des énantiomères ainsi obtenus est transformé en son fumarate par addition d'une solution d'acide fumarique dans l'isopropanol, puis cristallisation.
*Point de fusion : 187°C*
*Pouvoir rotatoire : α*_{*D*} = *-22,73° (eau, c = 1, λ = 589 nm)*

| *Microanalyse élémentaire :* | | | |
|---|---|---|---|
| | *C%* | *H%* | *N%* |
| *calculé* | *57,76* | *8,20* | *10,36* |
| *trouvé* | *57,47* | *8,21* | *10,15* |

### EXEMPLE 12 : (1β,2α,1'α)-2-[1'-Amino-2'-oxo-2'-(1-pyrrolidinyl)-éthyl]-cyclopentanol, hémifumarate

Le deuxième des énantiomères qui sont séparés à l'exemple 11 est transformé en son hémifumarate par addition d'une solution d'acide fumarique dans l'isopropanol, puis cristallisation.
*Point de fusion : 185°C*
*Pouvoir rotatoire : α*_{*D*} = *+22,95° (eau , c=1, λ* = 589 *nm)*

| *Microanalyse élémentaire :* | | | |
|---|---|---|---|
| | *C%* | *H%* | *N%* |
| *calculé* | *57,76* | *8,20* | *10,36* |
| *trouvé* | *57,47* | *8,24* | *10,15* |

### EXEMPLE 13 : (1α,2α,1'β)-2-[1'-Amino-2'-oxo-2'-(1-pyrrolidinyl)-éthyl]-cyclopentanol, hémifumarate

Le mélange racémique de l'exemple 4 est séparé par chromatographie HPLC chirale préparative sur colonne Chiralpack AD (éluant : n-heptane / éthanol / diéthylamine 80/20/1). Le premier des énantiomères ainsi obtenus est transformé en son hémifumarate par addition d'une solution d'acide fumarique dans l'isopropanol, puis cristallisation.
*Point de fusion : 180°C*
*Pouvoir rotatoire : α*_{*D*} *= +18,95° (eau, c=1, λ = 589 nm)*

| *Microanalyse élémentaire :* | | | |
|---|---|---|---|
| | *C%* | *H%* | *N%* |
| *calculé* | *57,76* | *8,20* | *10,36* |
| *trouvé* | *56,63* | *7,83* | *9,50* |

### EXEMPLE 14 : (1R*,2S*,1'R*)-2-[1'-Amino-2'-oxo-2'-(1-morpholinyl)-éthyl]-cyclopentanol, hémifumarate

Le produit attendu est obtenu selon le procédé décrit dans l'exemple 7, en remplaçant, au stade F, la pyrrolidine par la morpholine.
*Point de fusion : 192°C*

| *Microanalyse élémentaire :* | | | |
|---|---|---|---|
| | *C%* | *H%* | *N%* |
| *calculé* | *52,32* | *7,02* | *8,13* |
| *trouvé* | *53,49* | *7,49* | *9,41* |

### EXEMPLE 15 : (1R*,2S*,1'S*)-2-[1'-Amino-2'-oxo-2'-(1-morpholinyl)-éthyl]-cyclopentanol, hémifumarate

Le produit attendu est obtenu selon le procédé décrit dans les stades D à G de l'exemple 7 à partir de morpholine et du deuxième des diastéréoisomères qui sont séparés au stade C de l'exemple 7.

| *Microanalyse élémentaire :* | | | |
|---|---|---|---|
| | *C%* | *H%* | *N%* |
| *calculé* | *52,32* | *7,02* | *8,13* |
| *trouvé* | *52,83* | *7,11* | *8,77* |

### EXEMPLE 16 : (1R*,2S*,1'R*)-2-[1'-Amino-2'-oxo-2'-(1-azétidinyl)-éthyl]-cyclopentanol, hémifumarate

Le produit attendu est obtenu selon le procédé décrit dans l'exemple 7, en remplaçant, au stade F, la pyrrolidine par l'azétidine.
*Point de fusion : 208°C*

| *Microanalyse élémentaire :* | | | |
|---|---|---|---|
| | *C%* | *H%* | *N%* |
| *calculé* | *56,24* | *7,87* | *10,93* |
| *trouvé* | *56,25* | *7,84* | *10,66* |

### EXEMPLE 17 : (1R*,2S*,1'S*)-2-[1'-Amino-2'-oxo-2'-(1-azétidinyl)-éthyl]-cyclopentanol, hémifumarate

Le produit attendu est obtenu selon le procédé décrit dans les stades D à G de l'exemple 7 à partir d'azétidine et du deuxième des diastéréoisomères qui sont séparés au stade C de l'exemple 7.
*Point de fusion : 206°C*

| *Microanalyse élémentaire :* | | | |
|---|---|---|---|
| | *C%* | *H%* | *N%* |
| *calculé* | *56,24* | *7,87* | *10,93* |
| *trouvé* | *55,99* | *7,82* | *10,73* |

### EXEMPLE 18 : (1R*,2S*,1'R*)-2-[1'-Amino-2'-oxo-2'-(1-pipéridinyl)-éthyl]-cyclopentanol, hémifumarate

Le produit attendu est obtenu selon le procédé décrit dans l'exemple 7, en remplaçant, au stade F, la pyrrolidine par la pipéridine.
*Point de fusion : 188°C*

| *Microanalyse élementaire :* | | | |
|---|---|---|---|
| | *C%* | *H%* | *N%* |
| *calculé* | *59,14* | *8,51* | *9,85* |
| *trouvé* | *58,82* | *8,45* | *9,65* |

### EXEMPLE 19 : (1R*,2S*,1'S*)-2-[1'-Amino-2'-oxo-2'-(1-pipéridinyl)-éthyl]-cyclopentanol, hémifumarate

Le produit attendu est obtenu selon le procédé décrit dans les stades D à G de l'exemple 7 à partir de pipéridine et du deuxième des diastéréoisomères qui sont séparés au stade C de l'exemple 7.
*Point de fusion : 130°C*

| *Microanalyse élémentaire :* | | | |
|---|---|---|---|
| | *C%* | *H%* | *N%* |
| *calculé* | *59,14* | *8,51* | *9,85* |
| *trouvé* | *59,02* | *8,54* | *9,68* |

### EXEMPLE 20 : (1R*,2S*,1'R*)-2-[1'-Amino-2'-oxo-2'-(2-aza-bicyclo[3.3.0]octan-2-yl)-éthyl]-cyclopentanol, hémifumarate

Le produit attendu est obtenu selon le procédé décrit dans l'exemple 7, en remplaçant, au stade F, la pyrrolidine par le 2-aza-bicyclo[3.3.0]-octane.
*Point de fusion : 194°C*

| *Microanalyse élémentaire :* | | | |
|---|---|---|---|
| | *C%* | *H%* | *N%* |
| *calculé* | *61,91* | *8,44* | *9,03* |
| *trouvé* | *61,65* | *8,36* | *8,90* |

### EXEMPLE 21 : (1R*,2S*,1'S*)-2-[1'-Amino-2'-oxo-2'-(2-aza-bicyclo[3.3.0]octan-2-yl)-éthyl]-cyclopentanol, hémifumarate

Le produit attendu est obtenu selon le procédé décrit dans les stades D à G de l'exemple 7 à partir de 2-aza-bicyclo[3.3.0]-octane et du deuxième des diastéréoisomères qui sont séparés au stade C de l'exemple 7.
*Point de fusion : 185°C*

| *Microanalyse élémentaire :* | | | |
|---|---|---|---|
| | *C%* | *H%* | *N%* |
| *calculé* | *61,91* | *8,44* | *9,03* |
| *trouvé* | *61,52* | *8,33* | *8,87* |

### EXEMPLE 22 : (1R*,2S*,1'R*)-2-[1'-Amino-2'-oxo-2'-(1,3-thiazolidin-3-yl)-éthyl]-cyclopentanol, hémifumarate

### Stade A : (1R*,2S*,1'R*)-[(Tert-butyloxycarbonyl)-amino]-(2-hydroxycyclopentyl)-acétate d 'éthyle

A 10 mmoles du composé décrit dans le stade C de l'exemple 7 en solution dans le tert-butanol est ajoutée une solution de soude 1N (11 mmoles) et du dicarbonate de di-tert-butyle (11 mmoles). Après 1 heure d'agitation, les solvants sont évaporés, le résidu est repris par de l'acétate d'éthyle, la phase organique est lavée, séchée et évaporée pour conduire au produit attendu.

### Stade B : (1R*,2S*,1'R*)-2-[1'-{(Tert-butyloxycarbonyl)-amino}-2'-oxo-2'-(1,3-thiazolidin-3-yl)-éthyl]-cyclopentanol

Le produit attendu est obtenu selon le procédé décrit dans les stades E et F de l'exemple 7, en remplaçant, au stade F, la pyrrolidine par la 1,3-thiazolidine.

### Stade C : (1R*,2S*,1'R*)-2-[1'-Amino -2'-oxo-2'-(1,3-thiazolidin-3-yl)-éthyl]-cyclopentanol

Le produit obtenu au stade précédent est déprotégé par le perchlorate de magnésium, au reflux de l'acétonitrile, selon le procédé décrit dans Chem. Comm. 1999, pp. 1809-1810, pour conduire au produit attendu.

### Stade D : (1R*,2S*,1'R*)-2-[1'-Amino-2'-oxo-2'-(1,3-thiazolidin-3-yl)-éthyl]-cyclopentanol, hémifumarate

Le produit obtenu au stade précédent est transformé en son hémifumarate par addition d'une solution d'acide fumarique dans l'isopropanol, puis cristallisation.
*Point de fusion : 207°C*

| *Microanalyse élémentaire :* | | | | |
|---|---|---|---|---|
| | *C%* | *H%* | *N%* | *S%* |
| *calculé* | *49,98* | *6,99* | *9,71* | *11,12* |
| *trouvé* | *49,68* | *7,06* | *9,51* | *11,33* |

### EXEMPLE 23 : (1R*,2S*,1'S*)-2-[1'-Amino-2'-oxo-2'-(1,3-thiazolidin-3-yl)-éthyl]-cyclopentanol, fumarate

Le produit attendu est obtenu selon le procédé décrit dans l'exemple 22 à partir de 1,3-thiazolidine et du deuxième des diastéréoisomères qui sont séparés au stade C de l'exemple 7.
*Point de fusion : 190°C*

| *Microanalyse élémentaire :* | | | | |
|---|---|---|---|---|
| | *C%* | *H%* | *N%* | *S%* |
| *calculé* | *48,54* | *6,40* | *8,09* | *9,26* |
| *trouvé* | *48,86* | *6,49* | *7,85* | *9,20* |

### EXEMPLE 24 : (1R*,2S*,1'R*)-2-[1'-Amino-2'-oxo-2'-(1-pyrrolidinyl)-éthyl]-cyclohexanol, fumarate

### Stade A : (1R*,2S*,1'R*)-2-[1'-Amino-2'-oxo-2'-(1-pyrrolidinyl)-éthyl]-cyclohexanol

Le produit attendu est obtenu selon le procédé décrit dans le stade C de l'exemple 1, à partir du deuxième des diastéréoisomères obtenus au stade B de l'exemple 1.

### Stade B : (1R*,2S*,1'R*)-2-[1'-Amino-2'-oxo-2'-(1-pyrrolidinyl)-éthyl]-cyclohexanol, fumarate

Le produit attendu est obtenu selon le procédé décrit dans le stade D de l'exemple 1, à partir du composé obtenu au stade précédent.
*Point de fusion : 174°C*

| *Microanalyse élémentaire :* | | | |
|---|---|---|---|
| | *C%* | *H%* | *N%* |
| *calculé* | *56,13* | *7, 65* | *8,18* |
| *trouvé* | *55,86* | *7, 60* | *8,16* |

### EXEMPLE 25 : (1α,2β,1'α)-2-[1'-Amino-2'-oxo-2'-(1-pyrrolidinyl)-éthyl]-cyclohexanol, fumarate

Le mélange racémique obtenu au stade A de l'exemple 24 est séparé par chromatographie HPLC chirale préparative (éluant : n-heptane / éthanol / diéthylamine 90/10/1). Le premier des énantiomères obtenus est transformé en son fumarate par réaction avec une solution éthanolique d'acide fumarique, filtration puis séchage, pour conduire au produit attendu sous la forme d'un solide blanc.
*Point de fusion : 165°C*

| *Microanalyse élémentaire :* | | | |
|---|---|---|---|
| | *C%* | *H%* | *N%* |
| *calculé* | *56,13* | *7,65* | *8,18* |
| *trouvé* | *56,21* | *7,50* | *8,27* |

*Pouvoir rotatoire :α*_{*D*} = *+33,17° (eau, c=1, λ* = 589 *nm)*

### EXEMPLE 26 : (1β,2α,1'β)-2-[1'-Amino-2'-oxo-2'-(1-pyrrolidinyl)-éthyl]-cyclohexanol, fumarate

Le deuxième des énantiomères obtenus à l'exemple 25 est transformé en son fumarate par réaction avec une solution éthanolique d'acide fumarique, filtration puis séchage, pour conduire au produit attendu sous la forme d'un solide blanc.

| *Microanalyse élémentaire :* | | | |
|---|---|---|---|
| | *C%* | *H%* | *N%* |
| *calculé* | *56,13* | *7,65* | *8,18* |
| *trouvé* | *55,88* | *7,65* | *8,19* |

*Pouvoir rotatoire : α*_{*D*} *= -33,27° (eau , c=1, λ = 589 nm)*

### EXEMPLE 27 : (1R*,2R*,1'S*)-2-[1'-Amino-2'-oxo-2'-(1-pyrrolidinyl)-éthyl]-cyclohexanol, fumarate

### Stade A : (3S*,3aR*,7aR*)-3-Amino-hexahydro-1-benzofuran-2(3H)-one

Le composé obtenu au stade A de l'exemple 1 est soumis à une réaction de Mitsunobu, selon le procédé décrit dans Synthesis 1981, pp. 1 à 28.
Le produit ainsi obtenu est purifié par chromatographie sur silice, puis les diastéréoisomères sont séparés par HPLC préparative. Le produit attendu est le premier des diastéréoisomères ainsi séparés.

### Stade C: (1R*,2R*,1'S*)-2-[1'-Amino-2'-oxo-2'-(1-pyrrolidinyl)-éthyl]-cyclohexanol, fumarate

Le produit attendu est obtenu selon le procédé décrit dans les stades C et D de l'exemple 1, à partir du composé obtenu au stade précédent.
*Point de fusion : 198°C*

| *Microanalyse élémentaire :* | | | |
|---|---|---|---|
| | *C%* | *H%* | *N%* |
| *calculé* | *56,13* | *7,65* | *8,18* |
| *trouvé* | *55,92* | *7,62* | *8,14* |

### EXEMPLE 28 : (1R*,2R*,1'R*)-2-[1'-Amino-2'-oxo-2'-(1-pyrrolidinyl)-éthyl]-cyclohexanol, fumarate

Le produit attendu est obtenu selon le procédé décrit dans les stades C et D de l'exemple 1, à partir du deuxième des diastéréoisomères séparés au stade A de l'exemple 27.
*Point de fusion : 180°C*

| *Microanalyse élémentaire :* | | | |
|---|---|---|---|
| | *C%* | *H%* | *N%* |
| *calculé* | *56,13* | *7,65* | *8,18* |
| *trouvé* | *55,83* | *7,60* | *8,11* |

### EXEMPLE 29 : (1R*,2S*,1'R*)-2-[1'-Amino-2'-oxo-2'-(2,5-dihydro-1H-pyrrol-1-yl)-éthyl]-cyclohexanol, fumarate

Le produit attendu est obtenu selon le procédé décrit dans les stades C et D de l'exemple 1, à partir de 2,5-dihydro-1*H*-pyrrole et du deuxième des diastéréoisomères obtenus au stade B de l'exemple 1.

| *Microanalyse élementaire :* | | | |
|---|---|---|---|
| | *C%* | *H%* | *N%* |
| *calculé* | *56,46* | *7,11* | *8,23* |
| *trouvé* | *56,06* | *6,77* | *8,21* |

### EXEMPLE 30 : (1R*,2S*,1'R*)-2-[1'-Amino-2'-oxo-2'-(1,3-thiazolidin-3-yl)-éthyl]-cyclohexanol, fumarate

### Stade A : (1R*,2S*,1'R*)-2-[1'-Amino-2'-oxo-2'-(1,3-thiazolidin-3-yl)-éthyl]-cyclohexanol

Le produit attendu est obtenu selon le procédé décrit dans le stade C de l'exemple 1, à partir de 1,3-thiazolidine et du deuxième des diastéréoisomères obtenus au stade B de l'exemple 1.

### Stade B : (1R*,2S*,1'R*)-2-[1'-Amino-2'-oxo-2'-(1,3-thiazolidin-3-yl)-éthyl]-cyclohexanol, fumarate

Le produit attendu est obtenu selon le procédé décrit dans le stade D de l'exemple 1, à partir du composé obtenu au stade précédent.
*Point de fusion : 195°C*

| *Microanalyse élémentaire :* | | | | |
|---|---|---|---|---|
| | *C%* | *H%* | *N%* | *S%* |
| *calculé* | *49,99* | *6,71* | *7,77* | *8,90* |
| *trouvé* | *50,16* | *6,72* | *7,55* | *8,88* |

### EXEMPLE 31 : (1α,2β,1'α)-2-[1'-Amino-2'-oxo-2'-(1,3-thiazolidin-3-yl)-éthyl]-cyclohexanol, fumarate

Le mélange racémique obtenu au stade A de l'exemple 30 est séparé par chromatographie HPLC chirale préparative (éluant : éthanol / diéthylamine 1000/1). Le premier des énantiomères obtenus est transformé en son fumarate par réaction avec une solution éthanolique d'acide fumarique, filtration puis séchage, pour conduire au produit attendu sous la forme d'un solide blanc.
*Point de fusion : 190°C*

| *Microanalyse élémentaire :* | | | | |
|---|---|---|---|---|
| | *C%* | *H%* | *N%* | *S%* |
| *calculé* | *49,99* | *6,71* | *7,77* | *8,90* |
| *trouvé* | *49,79* | *6,74* | *7,57* | *8,86* |

### EXEMPLE 32 : (1β,2α,1'β)-2-[1'-Amino-2'-oxo-2'-(1,3-thiazolidin-3-yl)-éthyl]-cyclohexanol, fumarate

Le deuxième des énantiomères obtenus à l'exemple 31 est transformé en son fumarate par réaction avec une solution éthanolique d'acide fumarique, filtration puis séchage, pour conduire au produit attendu sous la forme d'un solide blanc.
*Point de fusion : 188°C*

| *Microanalyse élémentaire :* | | | | |
|---|---|---|---|---|
| | *C%* | *H%* | *N%* | *S%* |
| *calculé* | *49,99* | *6,71* | *7,77* | *8,90* |
| *trouvé* | *50,21* | *6,81* | *7,67* | *9,14* |

*Pouvoir rotatoire : α*_{*D*} *= +32,11*° *(eau*, *c=1, λ = 589 nm)*

### EXEMPLE 33 : (1R*,2S*,1'R*)-2-[1'-Amino-2'-oxo-2'-(1-pyrrolidinyl)-éthyl]-cycloheptanol, fumarate

### Stade A : (1R*,2S*,1'RS)-Amino-(2-hydroxycycloheptyl)-acétate d'éthyle

Le produit attendu est obtenu selon le procédé décrit dans les stades A et B de l'exemple 7, en remplaçant, au stade A, l'oxyde de cyclopentène par l'oxyde de cycloheptène.

### Stade B : (1R*,2S*,1'R*)-Amino-(2-hydroxycycloheptyl)-acétate d'éthyle

Le résidu obtenu au stade précédent est chromatographié sur silice (éluant: dichlorométhane/méthanol 90/10). Le produit attendu est le premier des 2 diastéréoisomères ainsi séparés.

### Stade C : (1R*,2S*,1'R*)-2-[1'-Amino-2-oxo-2-(1-pyrrolidinyl)-éthyl]-cycloheptanol, fumarate

Le produit attendu est obtenu selon le procédé décrit dans les stades D à G de l'exemple 7, à partir du composé obtenu au stade précédent.
*Point de fusion : 178°C*

| *Microanalyse élémentaire :* | | | |
|---|---|---|---|
| | *C%* | *H%* | *N%* |
| *calculé* | *57,29* | *7,92* | *7,86* |
| *trouvé* | *57,03* | *7,93* | *7,86* |

### EXEMPLE 34 : (1R*,2S*,1'S*)-2-[1'-Amino-2'-oxo-2'-(1,3-thiazolidin-3-yl)-éthyl]-cycloheptanol, fumarate

Le produit attendu est obtenu selon le procédé décrit dans les stades D à G de l'exemple 7 à partir du deuxième des diastéréoisomères qui sont séparés au stade B de l'exemple 33.

### EXEMPLE 35 : (1R*,2S*,1'S*)-2-[1'-Amino-2'-oxo-2'-(N,N-diéthylamino)-éthyl]-cyclopentanol, fumarate

Le produit attendu est obtenu selon le procédé décrit dans les stades D à G de l'exemple 7 à partir de N,N-diéthylamine et du deuxième des diastéréoisomères qui sont séparés au stade C de l'exemple 7.
*Point de fusion : 108°C*

| *Microanalyse élémentaire :* | | | |
|---|---|---|---|
| | *C%* | *H%* | *N%* |
| *calculé* | *54,53* | *7,93* | *8,48* |
| *trouvé* | *54,10* | *7,86* | *8,57* |

### EXEMPLE 36 : (1α,2β,1'α)-2-[1'-Amino-2'-oxo-2'-(1,3-thiazolidin-3-yl)-éthyl]-cyclopentanol, fumarate

Le mélange racémique obtenu au stade C de l'exemple 22 est séparé par chromatographie HPLC chirale préparative. Le premier des énantiomères obtenus est transformé en son fumarate par réaction avec une solution éthanolique d'acide fumarique, filtration puis séchage, pour conduire au produit attendu.

### EXEMPLE 37 : (1β,2α,1'β)-2-[1'-Amino-2'-oxo-2'-(1,3-thiazolidin-3-yl)-éthyl]-cyclopentanol, fumarate

Le deuxième des énantiomères obtenus à l'exemple 36 est transformé en son fumarate par réaction avec une solution éthanolique d'acide fumarique, filtration puis séchage, pour conduire au produit attendu.

### ETUDE PHARMACOLOGIQUE DES COMPOSES DE L'INVENTION

### EXEMPLE 38 : Test fonctionnel de surcharge au glucose par voie orale

Des rats Zucker mâles obèses (homozygotes fa/fa), âgés de 10 à 12 semaines subissent sous anesthésie générale (Pentobarbital sodique) une implantation d'un cathéter (silastic) au niveau de la veine jugulaire droite, 24 heures avant l'épreuve fonctionnelle de surcharge au glucose par voie orale (OGTT). Après 18 heures de jeûne, les animaux éveillés et isolés en cages individuelles reçoivent par intubation oesophagienne 1 g/kg d'une solution à 40 % dans l'eau de D(+)-glucose. L'administration des produits à tester (25, 10, 5 et 1 mg/kg) se fait soit en même temps que le glucose soit avant (-10, -20 min...). Des prélèvements sanguins sont effectués par l'intermédiaire du cathéter veineux aux temps (-20, -10) 0, 10, 20, 40 et 60 min après la surcharge en glucose, permettant le suivi de l'évolution de l'activité de l'insulinémie et de la glycémie.

Les composés de l'invention normalisent l'insulinémie et la glycémie à des doses comprises entre 1 et 25 mg/kg p.o.

### EXEMPLE 39 : Composition pharmaceutique

| Formule de préparation pour 1000 comprimés dosés à 10 mg | |
|---|---|
| Composé de l'exemple 1 | 10 g |
| Hydroxypropylcellulose | 2 g |
| Amidon de blé | 10g |
| Lactose | 100 g |
| Stéarate de magnésium | 3 g |
| Talc | 3 g |

## Revendications

1. Composé de formule (I) : 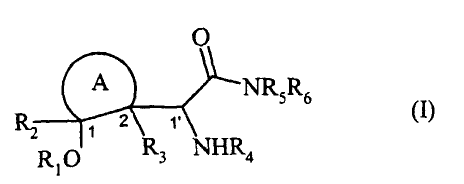 dans laquelle : 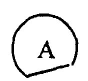
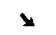 représente un cycle carboné saturé de 4 à 8 chaînons, éventuellement substitué par un ou plusieurs groupements alkyle (C₁-C₆) linéaire ou ramifié,
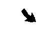 R₁ et R₄, identiques ou différents, représentent chacun un atome d'hydrogène ou un groupement acyle (C₁-C₆) linéaire ou ramifié,
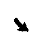 R₂ et R₃, identiques ou différents, représentent chacun un atome d'hydrogène ou un groupement alkyle (C₁-C₆) linéaire ou ramifié,
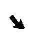 R₅ et R₆, identiques ou différents, représentent chacun un atome d'hydrogène ou un groupement alkyle (C₁-C₆)linéaire ou ramifié,
ou bien R₅ et R₆ forment ensemble, avec l'atome d'azote qui les porte, un hétérocycle azoté éventuellement substitué par un ou plusieurs groupements, identiques ou différents, choisis parmi les groupements cyano, CO₂R₇ (dans lequel R₇ représente un atome d'hydrogène ou un groupement alkyle (C₁-C₆) linéaire ou ramifié), COR₇ (dans lequel R₇ est tel que défini précédemment), nitro, CONR₈ₐR_{8b} (dans lequel R₈ₐ et R_{8b}, identiques ou différents, représentent chacun un atome d'hydrogène ou un groupement alkyle (C₁-C₆) linéaire ou ramifié, ou bien R₈ₐ et R_{8b} forment ensemble un hétérocycle azoté), S(O)ₙR₉ (dans lequel n représente 1, 2 ou 3, et R₉ représente un atome d'hydrogène, un groupement alkyle (C₁-C₆) linéaire ou ramifié ou aryle), ou PO₃R₁₀ₐR_{10b} (dans lequel R₁₀ₐ et R_{10b}, identiques ou différents, représentent chacun un atome d'hydrogène ou un groupement alkyle (C₁-C₆) linéaire ou ramifié),
ses stéréoisomères, ainsi que ses sels d'addition à un acide pharmaceutiquement acceptable,
étant entendu que par hétérocycle azoté, on entend un groupement saturé, mono- ou bicyclique, éventuellement ponté, comportant de 5 à 12 chaînons et contenant un, deux ou trois hétéroatomes, l'un de ces hétéroatomes étant l'atome d'azote, et le ou les hétéroatomes supplémentaires éventuellement présents étant choisis parmi les atomes d'oxygène, d'azote ou de soufre,
et par groupement aryle, on entend phényle, biphénylyle, naphtyle, ou tétrahydronaphtyle, chacun de ces groupements étant éventuellement substitué par un ou plusieurs atomes ou groupements, identiques ou différents, choisis parmi les atomes d'halogène et les groupements alkyle (C₁-C₆) linéaire ou ramifié, hydroxy, alkoxy (C₁-C₆) linéaire ou ramifié, polyhalogénoalkyle (C₁-C₆) linéaire ou ramifié, nitro ou alkylènedioxy (C₁-C₂).

2. Composé de formule (I) selon la revendication 1 tel que 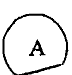 représente un cycle carboné de 5 ou 6 chaînons, ses stéréoisomères, ainsi que ses sels d'addition à un acide pharmaceutiquement acceptable.

3. Composé de formule (I) selon l'une quelconque des revendications 1 ou 2 tel que R₅ et R₆ forment ensemble un hétérocycle azoté éventuellement substitué, ses stéréoisomères, ainsi que ses sels d'addition à un acide pharmaceutiquement acceptable.

4. Composé de formule (I) selon la revendication 3 tel que R₅ et R₆ forment ensemble une pyrrolidine ou une thiazolidine éventuellement substituées, ses stéréoisomères, ainsi que ses sels d'addition à un acide pharmaceutiquement acceptable.

5. Composé de formule (I) selon la revendication 1 qui est le (1R,2S,1'S)-2-[1'-amino-2'-oxo-2'-(1-pyrrolidinyl)-éthyl]-cyclohexanol, son énantiomère (1S,2R,1'R), ainsi que leurs sels d'addition à un acide pharmaceutiquement acceptable.

6. Composé de formule (I) selon la revendication 1 qui est le (1R*,2R*,1'R*)-2-[1'-amino-2'-oxo-2'-(1-pyrrolidinyl)-éthyl]-cyclopentanol, ainsi que ses sels d'addition à un acide pharmaceutiquement acceptable, étant entendu que par composé (1R*,2R*,1'R*), on entend mélange racémique des 2 énantiomères de configurations absolues (1R,2R,1'R) et (1S,2S,1'S).

7. Composé de formule (I) selon la revendication 1 qui est le (1R,2S,1'R)-2-[1'-amino-2'-oxo-2'-(1-pyrrolidinyl)-éthyl]-cyclopentanol, son énantiomère (1S,2R,1'S), ainsi que leurs sels d'addition à un acide pharmaceutiquement acceptable.

8. Composé de formule (I) selon la revendication 1 qui est le (1R,2S,1'S)-2-[1'-amino-2'-oxo-2'-(1-pyrrolidinyl)-éthyl]-cyclopentanol, son énantiomère (1S,2R,1'R), ainsi que leurs sels d'addition à un acide pharmaceutiquement acceptable.

9. Composé de formule (I) selon la revendication 1 qui est le (1R,2S,1'R)-2-[1'-amino-2'-oxo-2'-(1-pyrrolidinyl)-éthyl]-cyclohexanol, son énantiomère (1S,2R,1'S), ainsi que leurs sels d'addition à un acide pharmaceutiquement acceptable.

10. Composé de formule (I) selon la revendication 1 qui est le (1R,2S,1'R)-2-[1'-amino-2'-oxo-2'-(1,3-thiazolidin-3-yl)-éthyl]-cyclohexanol, son énantiomère (1S,2R,1'S), ainsi que leurs sels d'addition à un acide pharmaceutiquement acceptable

11. Procédé de préparation des composés de formule (I) selon la revendication 1 **caractérisé en ce que** l'on met en réaction le chlorure de chloroacétyle avec le composé de formule (II) :
HNR₅R₆ (II)
dans laquelle R₅ et R₆ sont tels que définis dans la formule (I), pour conduire au composé de formule (III) : 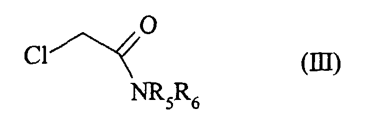 dans laquelle R₅ et R₆ sont tels que définis précédemment,
que l'on transforme en amine de formule (IV) : 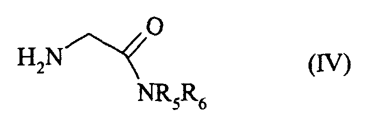 dans laquelle R₅ et R₆ sont tels que définis précédemment,
que l'on met en réaction avec le benzaldéhyde, pour conduire au composé de formule (V) : 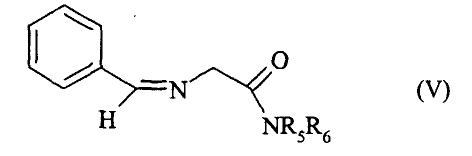 dans laquelle R₅ et R₆ sont tels que définis précédemment,
que l'on met en réaction avec le composé de formule (VI) : 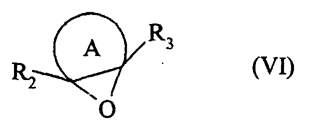 dans laquelle 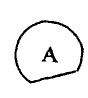 , R₂ et R₃ sont tels que définis dans la formule (I),
pour conduire après acylation éventuelle de la fonction hydroxy au composé de formule (VII), qui est de configuration relative trans : 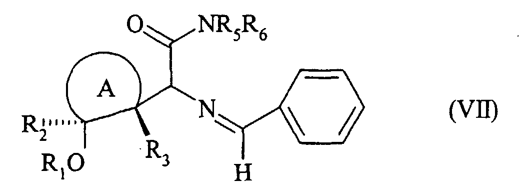 dans laquelle 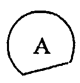 , R₁, R₂, R₃, R₅ et R₆ sont tels que défmis précédemment,
que l'on transforme, par hydrolyse suivie, lorsqu'on le souhaite, d'une acylation de la fonction amino, au composé de formule (Ia) de configuration relative trans, cas particulier des composés de formule (I) : 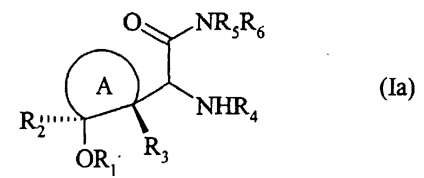 dans laquelle 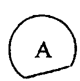 , R₁, R₂, R₃, R₄, R₅ et R₆ sont tels que définis précédemment,
que l'on transforme, lorsqu'on le souhaite, en composé de formule (Ib) de configuration relative cis, cas particulier des composés de formule (I) : 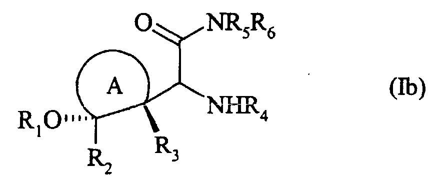 dans laquelle 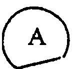 , R₁, R₂, R₃, R₄, R₅ et R₆ sont tels que définis précédemment,
composés de formules (Ia) et (Ib) dont on sépare les stéréoisomères, lorsqu'on le souhaite, par des techniques classiques de séparation, que l'on purifie, le cas échéant, par des méthodes classiques de purification, et que l'on transforme, lorsqu'on le souhaite, en leurs sels d'addition à un acide pharmaceutiquement acceptable.

12. Composition pharmaceutique contenant comme principe actif un composé selon l'une quelconque des revendications 1 à 10, en combinaison avec un ou plusieurs véhicules inertes, non toxiques et pharmaceutiquement acceptables.

13. Composition pharmaceutique selon la revendication 12 utile en tant que médicament dans le traitement de l'intolérance au glucose et des maladies associées à une hyperglycémie telles que le diabète de type II ou l'obésité.

14. Composition pharmaceutique selon la revendication 12 utile en tant que médicament antidiabétique.

## Patentansprüche

1. Verbindung der Formel (I): 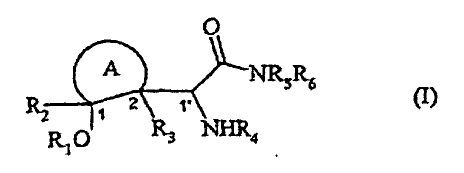 in der: 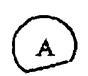
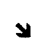 einen gesättigten Kohlenstoffring mit 4 bis 8 Kettengliedern, der gegebenenfalls durch eine oder mehrere geradkettige oder verzweigte (C₁-C₆)-Alkylgruppen substituiert ist, bedeutet,
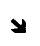 R₁ und R₄, die gleichartig oder verschieden sind, jeweils ein Wasserstoffatom oder eine geradkettige oder verzweigte (C₁-C₆)-Acylgruppe bedeuten,
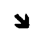 R₂ und R₃, die gleichartig oder verschieden sind, jeweils ein Wasserstoffatom oder eine geradkettige oder verzweigte (C₁-C₆)-Alkylgruppe bedeuten,
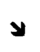 R₅ und R₆, die gleichartig oder verschieden sind, jeweils ein Wasserstoffatom oder eine geradkettige oder verzweigte (C₁-C₆)-Alkylgruppe bedeuten, oder R₅ und R₆ gemeinsam mit dem sie tragenden Stickstoffatom einen Stickstoff-haltigen Heterocyclus bilden, der gegebenenfalls durch eine oder mehrere gleichartige oder verschiedenartige Gruppen substituiert ist, die ausgewählt sind aus den folgenden Gruppen, Cyano, CO₂R₇ (worin R₇ ein Wasserstoffatom oder eine geradkettige oder verzweigte (C₁-C₆)-Alkylgruppe darstellt), COR₇ (worin R₇ die oben angegebenen Bedeutungen besitzt), Nitro, CONR₈ₐR_{8b} (worin R₈ₐ und R_{8b}, die gleichartig oder verschieden sind, jeweils ein Wasserstoffatom oder eine geradkettige oder verzweigte (C₁-C₆)-Alkylgruppe bedeuten oder R₈ₐ und R_{8b} gemeinsam einen Stickstoff-haltigen Heterocyclus bilden), S(O)ₙR₉ (worin n 1, 2 oder 3 bedeutet und R₉ ein Wasserstoffatom, eine geradkettige oder verzweigte (C₁-C₆)-Alkylgruppe oder eine Arylgruppe darstellt) oder PO₃R₁₀ₐR_{10b} (worin R₁₀ₐ und R_{10b}, die gleichartig oder verschieden sind, jeweils ein Wasserstoffatom oder eine geradkettige oder verzweigte (C₁-C₆)-Alkylgruppe bedeuten),
deren Stereoisomere sowie deren Additionssalze mit einer pharmazeutisch annehmbaren Säure,
mit der Maßgabe, daß man unter einem Stickstoff-haltigen Heterocyclus eine gesättigte, mono- oder bicyclische, gegebenenfalls überbrückte Gruppe mit 5 bis 12 Kettengliedern versteht, die ein, zwei oder drei Heteroatome aufweist, wobei eines dieser Heteroatome ein Stickstoffatom ist und wobei das oder die gegebenenfalls vorhandenen zusätzlichen Heteroatome ausgewählt sind aus Sauerstoff-, Stickstoff- oder Schwefelatomen,
und man unter einer Arylgruppe Phenyl, Biphenyl, Naphthyl oder Tetrahydronaphthyl versteht, wobei jede dieser Gruppen gegebenenfalls durch ein oder mehrere gleichartige oder verschiedenartige Atome oder Gruppen substituiert ist, ausgewählt aus Halogenatomen und geradkettigen oder verzweigten (C₁-C₆)-Alkylgruppen, Hydroxygruppen, geradkettigen oder verzweigten (C₁-C₆)-Alkoxygruppen, geradkettigen oder verzweigten (C₁-C₆)-Polyhalogenalkylgruppen, Nitrogruppen oder (C₁-C₆)-Alkylendioxygruppen.

2. Verbindung der Formel (I) nach Anspruch 1, worin 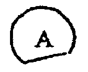 einen Kohlenstoffring mit 5 oder 6 Kettengliedern bedeutet, ihre Stereoisomeren sowie ihre Additionssalze mit einer pharmazeutisch annehmbaren Säure.

3. Verbindung der Formel (I) nach einem der Ansprüche 1 oder 2, worin R₅ und R₆ gemeinsam einen gegebenenfalls substituierten Stickstoff-haltigen Heterocyclus bilden, ihre Stereoisomeren sowie ihre Additionssalze mit einer pharmazeutisch annehmbaren Säure.

4. Verbindung der Formel (I) nach Anspruch 3, worin R₅ und R₆ gemeinsam einen gegebenenfalls substituierten Pyrrolidin- oder Thiazolidinring bilden, ihre Stereoisomeren sowie ihre Additionssalze mit einer pharmazeutisch annehmbaren Säure.

5. Verbindung der Formel (I) nach Anspruch 1, nämlich (1R,2S,1'S)-2-[1'-Amino-2'-oxo-2'-(1-pyrrolidinyl)-ethyl]-cyclohexanol, dessen (1S,2R,1'R)-Enantiomeres sowie dessen Additionssalze mit einer pharmazeutisch annehmbaren Säure.

6. Verbindung der Formel (I) nach Anspruch 1, nämlich (1R*,2R*,1'R*)-2-[1'-Amino-2'-oxo-2'-(1-pyrrolidinyl)-ethyl]-cyclopentanol sowie dessen Additionssalze mit einer pharmazeutisch annehmbaren Säure, mit der Maßgabe, daß man unter der (1R*,2R*,1'R*)-Verbindung die racemische Mischung der beiden Enantiomeren mit den absoluten Konfigurationen (1R,2R,1'R) und (1S,2S,1'S) versteht.

7. Verbindung der Formel (I) nach Anspruch 1, nämlich (1R,2S,1'R)-2-[1'-Amino-2'-oxo-2'-(1-pyrrolidinyl)-ethyl]-cyclopentanol, dessen (1S,2R,1'S)-Enantiomeres sowie deren Additionssalze mit einer pharmazeutisch annehmbaren Säure.

8. Verbindung der Formel (I) nach Anspruch 1, nämlich (1R,2S,1'S)-2-[1'-Amini-2'-oxo-2'-(1-pyrrolidinyl)-ethyl]-cyclopentanol, dessen (1S,2R,1'R)-Enantiomeres sowie deren Additionssalze mit einer pharmazeutisch annehmbaren Säure.

9. Verbindung der Formel (I) nach Anspruch 1, nämlich (1R,2S,1'R)-2-[1'-Amino-2'-oxo-2'-(1-pyrrolidinyl)-ethyl]-cyclohexanol, dessen (1S,2R,1'S)-Enantiomeres sowie deren Additionssalze mit einer pharmazeutisch annehmbaren Säure.

10. Verbindung der Formel (I) nach Anspruch 1, nämlich (1R,2S,1'R)-2-[1'-Amino-2'-oxo-2'-(1,3-thiazolidin-3-yl)-ethyl]-cyclohexanol, dessen (1S,2R,1'S)-Enantiomeres sowie deren Additionssalze mit einer pharmazeutisch annehmbaren Säure.

11. Verfahren zur Herstellung der Verbindungen der Formel (I) nach Anspruch 1, **dadurch gekennzeichnet, daß** man Chloracetylchlorid mit der Verbindung der Formel (II):
HNR₅R₆ (II)
in der R₅ und R₆ die bezüglich der Formel (I) angegebenen Bedeutungen besitzen, umsetzt
zur Bildung der Verbindung der Formel (III): 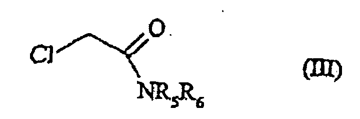 in der R₅ und R₆ die oben angegebenen Bedeutungen besitzen, welche man in das Amin der Formel (IV) umwandelt: 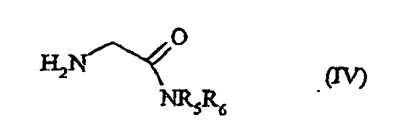 in der R₅ und R₆ die oben angegebenen Bedeutungen besitzen, welches man mit Benzaldehyd umsetzt zur Bildung der Verbindung der Formel (V): 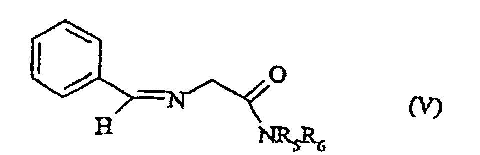 in der R₅ und R₆ die oben angegebenen Bedeutungen besitzen, welche man mit der Verbindung der Formel (VI) umsetzt: 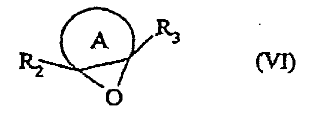 in der 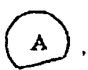 R₂ und R₃ die bezüglich der Formel (I) angegebenen Bedeutungen besitzen,
so daß man nach der eventuellen Acylierung der Hydroxyfunktion die Verbindung der Formel (VII) in der relativen trans-Konfiguration erhält: 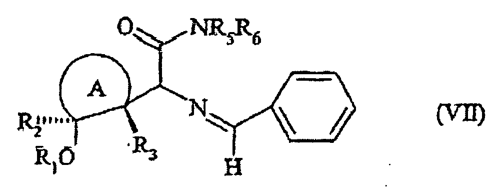 in der 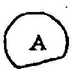 , R₁, R₂, R₃, R₅ und R₆ die oben angegebenen Bedeutungen besitzen,
welche man durch Hydrolyse, gewünschtenfalls gefolgt von einer Acylierung der Aminofunktion, in die Verbindung der Formel (Ia) in der relativen trans-Konfiguration umwandelt, einem Sonderfall der Verbindungen der Formel (I): 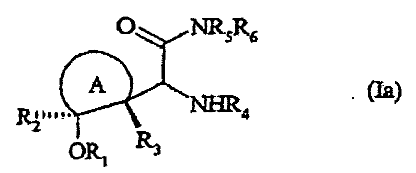 in der 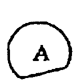 , R₁, R₂, R₃, R₄, R₅ und R₆ die oben angegebenen Bedeutungen besitzen,
welche man gewünschtenfalls in die Verbindung der Formel (Ib) in der relativen cis-Konfiguration umwandelt, einem Sonderfall der Verbindungen der Formel (I): 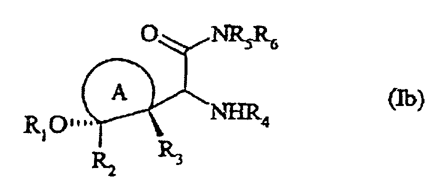 in der 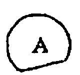 R₁, R₂, R₃, R₄, R₅ und R₆ die oben angegebenen Bedeutungen besitzen,
welche Verbindungen der Formel (Ia) und (Ib) man gewünschtenfalls mit Hilfe klassischer Trennmethoden in die Stereoisomeren auftrennt, gegebenenfalls mit Hilfe klassischer Reinigungsmethoden reinigt und gewünschtenfalls in ihre Additionssalze mit einer pharmazeutisch annehmbaren Säure umwandelt.

12. Pharmazeutische Zubereitung, enthaltend als Wirkstoff eine Verbindung nach einem der Ansprüche 1 bis 10 in Kombination mit einem oder mehreren inerten, nichttoxischen und pharmazeutisch annehmbaren Trägermaterialien.

13. Pharmazeutische Zubereitung nach Anspruch 12, nützlich als Arzneimittel bei der Behandlung der Glucoseunverträglichkeit und von Erkrankungen, die mit einer Hyperglykämie verknüpft sind, wie Diabetes Typ II oder der Fettsucht.

14. Pharmazeutische Zubereitung nach Anspruch 12, nützlich als antidiabetisches Arzneimittel.

## Claims

1. Compound of formula (I) : 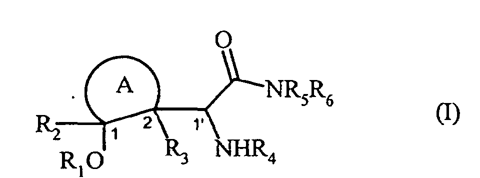 wherein: 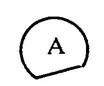
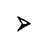 represents a saturated carbon ring having from 4 to 8 ring members, optionally substituted by one or more linear or branched (C₁-C₆)alkyl groups,
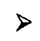 R₁ and R₄, which may be identical or different, each represents a hydrogen atom or a linear or branched (C₁-C₆)acyl group,
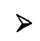 R₂ and R₃, which may be identical or different, each represents a hydrogen atom or a linear or branched (C₁-C₆)alkyl group,
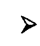 R₅ and R₆, which may be identical or different, each represents a hydrogen atom or a linear or branched (C₁-C₆)alkyl group,
or R₅ and R₆ together, with the nitrogen atom carrying them, form a nitrogen-containing heterocycle optionally substituted by one or more identical or different groups selected from the groups cyano, CO₂R₇ (wherein R₇ represents a hydrogen atom or a linear or branched (C₁-C₆)alkyl group), COR₇ (wherein R₇ is as defined hereinbefore), nitro, CONR₈ₐR_{8b} (wherein R₈ₐ and R_{8b}, which may be identical or different, each represents a hydrogen atom or a linear or branched (C₁-C₆)alkyl group or R₈ₐ and R_{8b} together form a nitrogen-containing heterocycle), S(O)ₙR₉ (wherein n represents 1, 2 or 3, and R₉ represents a hydrogen atom, a linear or branched (C₁-C₆)-alkyl group or an aryl group) and PO₃R₁₀ₐR_{10b} (wherein R₁₀ₐ and R_{10b}, which may be identical or different, each represents a hydrogen atom or a linear or branched (C₁-C₆)-alkyl group),
stereoisomers thereof, and also addition salts thereof with a pharmaceutically acceptable acid,
a nitrogen-containing heterocycle being understood as an optionally bridged, saturated mono- or bi-cyclic group having from 5 to 12 ring members and containing one, two or three hetero atoms, one of those hetero atoms being the nitrogen atom and the additional hetero atom or atoms optionally present being selected from the atoms oxygen, nitrogen and sulphur,
and an aryl group being understood as phenyl, biphenylyl, naphthyl or tetrahydronaphthyl, each of those groups optionally being substituted by one or more identical or different atoms or groups selected from the halogen atoms and the groups linear or branched (C₁-C₆)alkyl, hydroxy, linear or branched (C₁-C₆)alkoxy, linear or branched (C₁-C₆)polyhaloalkyl, nitro and (C₁-C₂)alkylenedioxy.

2. Compound of formule (I) according to claim 1, wherein 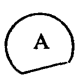 represents a carbon ring having 5 or 6 ring members, stereoisomers thereof, and also addition salts thereof with a pharmaceutically acceptable acid.

3. Compound of formula (I) according to either claim 1 or claim 2, wherein R₅ and R₆ together form an optionally substituted nitrogen-containing heterocycle, stereoisomers thereof, and also addition salts thereof with a pharmaceutically acceptable acid.

4. Compound of formula (I) according to claim 3, wherein R₅ and R₆ together form an optionally substituted pyrrolidine or an optionally substituted thiazolidine, stereoisomers thereof, and also addition salts thereof with a pharmaceutically acceptable acid.

5. Compound of formula (I) according to claim 1, which is (1R,2S,1'S)-2-[1'-amino-2'-oxo-2'-(1-pyrrolidinyl)ethyl]cyclohexanol, its (1S,2R,1'R) enantiomer, and also addition salts thereof with a pharmaceutically acceptable acid.

6. Compound of formula (I) according to claim 1, which is (1R*,2R*,1'R*)-2-[1'-amino-2'-oxo-2'-(1-pyrrolidinyl)ethyl]cyclopentanol, and also addition salts thereof with a pharmaceutically acceptable acid, there being understood by (1R*,2R*,1'R*) compound a racemic mixture of the 2 enantiomers having the absolute configurations (1R,2R,1'R) and (1S,2S,1'S).

7. Compound of formula (I) according to claim 1, which is (1R,2S,1'R)-2-[1'-amino-2'-oxo-2'-(1-pyrrolidinyl)ethyl]cyclopentanol, its (1S,2R,1'S) enantiomer, and also addition salts thereof with a pharmaceutically acceptable acid.

8. Compound of formula (I) according to claim 1, which is (1R,2S,1'S)-2-[1'-amino-2'-oxo-2'-(1-pyrrolidinyl)ethyl]cyclopentanol, its (1S,2R,1'R) enantiomer, and also addition salts thereof with a pharmaceutically acceptable acid.

9. Compound of formula (I) according to claim 1, which is (1R,2S,1'R)-2-[1'-amino-2'-oxo-2'-(1-pyrrolidinyl)ethyl]cyclohexanol, its (1S,2R,1'S) enantiomer, and also addition salts thereof with a pharmaceutically acceptable acid.

10. Compound of formula (I) according to claim 1, which is (1R,2S,1'R)-2-[1'-amino-2'-oxo-2'-(1,3-thiazolidin-3-yl)ethyl]cyclohexanol, its (1S,2R,1'S) enantiomer, and also addition salts thereof with a pharmaceutically acceptable acid.

11. Process for the preparation of compounds of formula (I) according to claim I, **characterised in that** chloroacetyl chloride is reacted with a compound of formula (II) :
HNR₅R₆ (II)
wherein R₅ and R₆ are as defined for formula (I),
to yield a compound of formula (III) : 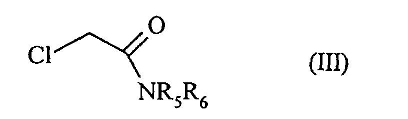 wherein R₅ and R₆ are as defined hereinbefore,
which is converted into an amine of formula (IV) : 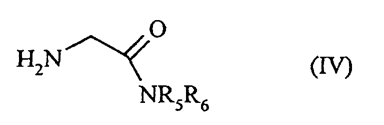 wherein R₅ and R₆ are as defined hereinbefore,
which is reacted with benzaldehyde to yield a compound of formula (V) : 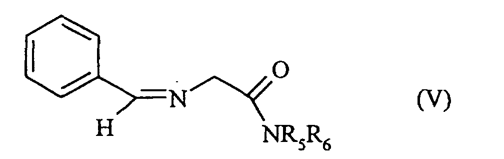 wherein R₅ and R₆ are as defined hereinbefore,
which is reacted with a compound of formula (VI) : 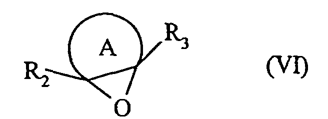 wherein 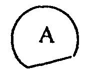 , R₂ and R₃ are as defined for formula (I),
to yield, after optional acylation of the hydroxy function, a compound of formula (VII), which is of the relative configuration trans: 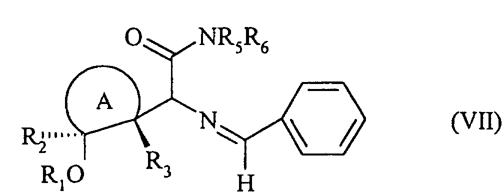 wherein 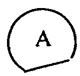 , R₁, R₂, R₃, R₅ and R₆ are as defined hereinbefore,
which is converted by hydrolysis followed, if desired, by acylation of the amino function, to yield a compound of formula (Ia) of the relative configuration trans, a particular case of the compounds of formula (I): 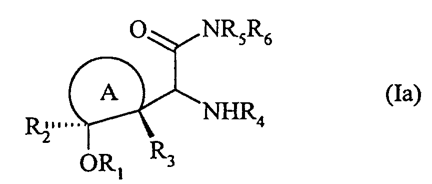 wherein 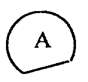 , R₁, R₂, R₃, R₄, R₅ and R₆ are as defined hereinbefore,
which is converted, if desired, into a compound of formula (Ib) of the relative configuration cis, a particular case of the compounds of formula (I) : 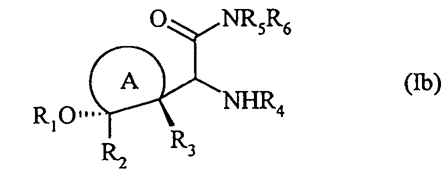 wherein 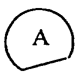 , R₁, R₂, R₃, R₄, R₅ and R₆ are as defined hereinbefore,
which compounds of formulae (Ia) and (Ib) are separated, if desired, into their stereoisomers by conventional separation techniques, are purified, if necessary, by conventional methods of purification, and are converted, if desired, into addition salts with a pharmaceutically acceptable acid.

12. Pharmaceutical composition comprising as active ingredient a compound according to any one of claims 1 to 10, in combination with one or more pharmaceutically acceptable, inert, non-toxic carriers.

13. Pharmaceutical composition according to claim 12 for use as a medicament in the treatment of glucose intolerance and of disorders associated with hyperglycaemia, such as type II diabetes or obesity.

14. Pharmaceutical composition according to claim 12 for use as an anti-diabetic medicament.
